# EUROPEAN PATENT SPECIFICATION

(11) **EP 3 831 437 B1**
(45) Date of publication and mention of the grant of the patent: **20.11.2024**
(21) Application number: 19306590.1
(22) Date of filing: 06.12.2019
(51) Int. Cl.: A61M 25/01

(54) **ANCHORING ELEMENTS FOR A STEERABLE DEVICE AND ASSEMBLY METHOD THEREOF**
VERANKERUNGSELEMENTE FÜR EINE LENKBARE VORRICHTUNG UND HERSTELLUNGSVERFAHREN
ÉLÉMENTS D'ANCRAGE POUR UN DISPOSITIF ORIENTABLE ET PROCÉDÉ DE FABRICATION

(43) Date of publication of application: 09.06.2021
(73) Proprietor: Basecamp Vascular, 75013 Paris (FR)
(72) Inventor: CAZENEUVE, Jean-Baptiste, 94200 Ivry-sur-Seine (FR); MAIANO, Camille, 29810 Brélès (FR)
(74) Representative: Icosa

(56) References cited:
- DE-A1- 102009 037 827
- US-A- 5 383 852
- US-A1- 2012 053 569
- US-A1- 2013 281 925
- US-A1- 2019 329 004

## Description

### FIELD OF INVENTION

The present invention relates to a steerable device, for instance, such device is used as a guide for a catheter or an endoscope for internally investigating a tubular element such as a pipe, a duct or an artery. The device of the invention is particularly suitable for use in the field of surgical investigation inside the body of a subject.

Even though the device is designed for surgical purpose, it can also be used in other areas needing non-destructive control or diagnostic such as pipeline systems for instance.

### BACKGROUND OF INVENTION

There is a wide range of applications where there is a need for using a device inside a pipe, a duct or a tube, for example for the placement of the distal part of a flexible tube in a specific location, for investigation or bringing pharmaceuticals, or functionality at a remote or hard-to-access location.

When displacing a flexible elongated device in the lumen of a pipe, duct or a tube, it is important for the user to be able to control carefully and precisely the movement and the placement of such device. Placement of such devices within pipes is a known technical issue in oil engineering or in motor engineering. Placement of devices within body tubes, such as for example through the ostium (of a vein, an artery, the gastro-intestinal track...etc), is also known in the medical field as being challenging.

In the medical field, using surgical or endovascular techniques, one can treat a lot of cardiovascular diseases causing death in the world. One of the pathologies encountered is the myocardial infarction along with peripheral vascular diseases. Through the last decades, the use of catheters and guidewires to reach pathological areas so as to deliver stents or balloons has emerged as an easy-to-implement solution. These endovascular techniques are less invasive compared to conventional surgeries. They come with a decreased recovery time and less post operation complications.

However, generally speaking, the surgeon skill and experience are a major success factor in the complex interventions while recent developments aim at facilitating navigation through complex anatomies as independently as possible from those surgeon skill and experience.

To do so, for example, it is known the application WO95/06494 that discloses a flexible elongated device comprising a flexible elongate member with proximal and distal extremities. A shape memory element disposed in the flexible elongate member and capable of assuming martensitic and austenitic states and having first and second portions. A layer of conductive material formed on at least one of said portions. The layer has a conductivity greater than that of the shape memory element. Electrical current is supplied to the shape memory element. The conductive layer serves to conduct current and shunts current flow around that portion of the shape memory element having the layer of conductive material thereon. This disclosure requires however a complex assembly with multiple inner connections inside the elongated device with a high risk of having a disassembly between the shape memory element and its associated inner knot.

So as to overcome such complexity, lower disassembly risks and improve manufacturability, it is also known the patent application WO2018167300 disclosing a an elongated steerable system for guiding a catheter or an endoscope, comprising: an elongated flexible member comprising, along at least a part of its longitudinal axis, at least one projection which, in transversal section, projects from the elongated flexible member; said at least one projection having a distal end and a proximal end, and defining, in transversal section, two lateral sides projecting from the elongated flexible member; and a wire; wherein said at least one projection comprises at least one transverse retaining and passing means near the distal end of said projection, said retaining and passing means extending transversely to the lateral sides of the projection; and the wire passes through said retaining and passing means alternatively from one lateral side of the projection to the other lateral side of the projection, wherein adherence between the wire and the retaining and passing means retains the wire ensuring the anchorage of the wire to said retaining and passing means.

Further steering systems for catheters are known from US 2019/0329004 and US2012/0053569.

These prior art devices are either complex and/or cumbersome and do not offer enough bending ability to navigate inside the lumen of a tube or pipe. The main objective of the invention is to provide a steerable device comprising actuators and fastening means that are easy to produce, compact and repeatable. According to the invention, the steerable device obtained is flexible enough to allow easy navigation inside a lumen, tube or pipe.

### SUMMARY

The invention is defined in independent device claim 1 and independent method claim 17. Further aspects of the invention are defined in dependent claims 2-16 and 18. This invention thus relates to a steerable device configured to be advanced in the lumen of a tubular element, said device comprising:
- a flexible axially elongated member having proximal and distal ends,
- at least one actuating means arranged alongside the periphery of said elongated member,
- at least one fastening means configured to fasten at least partially the at least one actuating means to the flexible elongated member distal end, said fastening means being in direct contact with the flexible elongated member,
- at least one anti-return means configured to keep the at least one actuating means from sliding alongside the periphery of the flexible elongated member distal end,
characterized in that the at least one anti-return means and the at least one fastening means are in axial abutting contact so as to prevent the at least one actuating means from sliding once actuated.

In a preferred embodiment, the at least one actuating means is made of a shape memory alloy such as NiTi alloy. Such alloys are used in the invention in surgical domain for their biocompatibility as well as their low density. The actuated bending motion is obtained by implementing electrical current that induces a phase transformation in the material through the Joule effect. Once the current no longer flows, the actuating means goes back to initial straight position thanks to the assembly spring back force. The higher the current, the higher the bending angle until a threshold.

In a preferred embodiment, the at least one actuating means comprises a spring or a wire that are more practical, lighter and more compact for a small diameter pipe, lumen or tube.

Preferably, the at least one flexible axially elongated member is wire or blade shaped. It may have a cross-section profile under a form selected from star, circular, semicircular, square, rectangular, triangle, pyramidal or any combinations thereof.

In another alternative, the at least one flexible axially elongated member is spring shaped. More preferably, the spring is a helicoidal spring.

Preferably, the at least one flexible axially elongated member is blade shaped so as to improve assembly and to have a preferred common bending plane for the actuators. In an alternative embodiment, the flexible axially elongated member is wire shaped.

In an alternative embodiment, the fastening means is a ligation around the flexible elongated member. Just like for the actuating means, ligations are easy to implement and reduce the spatial congestion.

In an alternative embodiment, the anti-return means is a glue filled tube, the actuating means going through said tube. The advantage of this configuration is that the actuating means are very solidly anchored while the tube end thickness serves as axial abutments for the fastening means.

In a variant, the anti-return means is a crimped tube, the actuating means going through said tube. The advantage of this alternative configuration is that the actuating means are also very solidly anchored while the tube end thickness serves as axial abutments for the fastening means.

Advantageously, another second anti-return means is made integral with the flexible elongated member so as to maintain the fastening means in place. Particularly, said anti-return means being a recess built into the flexible elongated member and configured to host the at least one fastening means. Preferably, the recess extends in a direction perpendicular to the longitudinal axis of the flexible elongated member. In this case with the anti-return means made integral with the flexible elongated member, the device according to the invention is easy to produce, compact, and presents a low disassembly risk for the anti-return means. In addition, the assembly robustness is improved.

In an exemplary embodiment, the anti-return means is made integral with the actuating means. In this other configuration also, the device according to the invention is easy to produce, compact, and presents a low disassembly risk for the anti-return means
In an exemplary embodiment, the at least one anti-return means is a loop encapsulating at least partially the at least one fastening means or said loop being an abutment for the at least one fastening means. The loop is obtained by modifying the shape of the actuating means. It is also possible to have a configuration where the at least one anti-return means is a knot, a weld or any local reinforcement located at the at least one actuating means distal end. These configurations add simplicity to the low disassembly risk.

Advantageously, the at least one anti-return means is a groove built into the actuating means and configured to host the at least one fastening means. This configuration adds simplicity to the low disassembly risk. Preferably, the groove extends in a direction perpendicular to the longitudinal axis of the flexible elongated member. The groove can be obtained by an indentation process.

In an advantageous embodiment, the at least one actuating means is inside an electrically isolating material such as a tube or a coating. The material has preferably an electrical and/or temperature isolation effect.

In another advantageous embodiment, the at least one actuating means is arranged alongside the external periphery of the elongated member. In this case, the device assembly is made easier.

In another advantageous embodiment, the at least one actuating means is arranged alongside the internal periphery of the elongated member. In this case, the external diameter is constant and the device according to the invention is less bulky.

The invention has also as an object an assembly method for steerable device according to the invention comprising the steps of:
- providing a flexible axially elongated member having proximal and distal ends,
- arranging at least one actuating means alongside the periphery of said elongated member,
- fastening the at least one actuating means to the flexible elongated member distal end with at least one fastening means,
- Providing at least one anti-return means configured to keep the at least one actuating means from sliding alongside the periphery of the flexible elongated member distal end, characterized in that the at least one anti-return means and the at least one fastening means are in axial abutting contact so as to prevent the at least one actuating means from sliding once actuated.

In a preferred embodiment, in the method according to the invention, the at least one actuating means is arranged alongside the external periphery of the elongated member. In this case, the device assembly is made easier.

In another advantageous embodiment, in the method according to the invention, the at least one actuating means is arranged alongside the internal periphery of the elongated member. In this case, the external diameter is constant and the device obtained according to the method of the invention is less bulky.

The assembly method according to the invention may be such that the at least one actuating means is a wire, the at least one fastening means is a ligation and the at least one anti-return means is obtained by increasing the contact pressure between the wire and the ligation , with said ligation going alternatively up and under the wire so as to ligate longitudinally partial parts of the wire with the flexible elongated member. This method is simple to implement and reduces spatial congestion for the device according to the invention.

### DEFINITIONS

In the present invention, the following terms have the following meanings:
- "About": is used herein to mean approximately, roughly, around, or in the region of. When the term "about" is used in conjunction with a numerical range, it modifies that range by extending the boundaries above and below the numerical values set forth. According to one embodiment, the term "about" preceding a figure means plus or less 10% of the value of said figure.
- "Actuator": can be any type of string, cable, wire, ribbon, tube or any set of those, capable of activating the body part to which it is fixed in order to trigger a function or to induce a bending of an area of the body part to which it is fixed. Actuators may be materials and devices that are able to change their shape (shape memory material) in response to changes in environmental conditions, temperature, and perform mechanical work, but this not an exclusive option. An actuator may convey energy. Most of the time, an actuator transforms the received energy into another type of energy. In one embodiment, the actuator receives an electrical current which is transformed into heat thanks to Joule effect, and upon action of the heat, contracts.
- "Catheter" is a tubular medical device for insertion into canals, vessels, passageways or body cavities for diagnostic, surgical or therapeutic purposes such as to permit injection/withdrawal of fluids, to keep passageways open, to inspect internal organs and tissues and to place medical tools into position for medical treatment within the body of an animal or of a human. In this invention, the term "catheter" encompasses any cannula or medical probe designed for insertion in a human or animal canal, vessel, passageway or body cavity.
- "to curvate": means to take the form of a curvature, to bend. Having a curvature or being curved is used in opposition to being straight. The term "curvature" means non-zero curvature. The curvature can be positive or negative.
- "Elongated": refers to an element such as a body, device, or a system that extends longitudinally.
- "Means": refers to elements or configurations in the context of the invention. "Flexible": refers to an object that may bend without breaking.
- "Anti-return means": refers to an element or a configuration that is able to keep another element to which it is connected, from sliding. In the case of a configuration, such configuration keeps the target element from sliding.

### BRIEF DESCRIPTION OF THE DRAWINGS

**Figures 1A and 1B** are illustrations of the device according to the invention with recesses in the flexible axially elongated member as anti-return means in top view and perspective views. The perspective view presents a different configuration for the flat wire actuating means which has smaller width.
**Figures 2A, 2B and 2C** are illustrations of the device according to the invention with a loop as anti-return means, such loop standing alone (2A), encapsulating partially the fastening means (2B) and encapsulating totally the fastening means (2C).
**Figures 3A and 3B** are illustrations of the device according to the invention with a knot as anti-return means, Fig. 3A shows one knot and Fig. 3B, two knots.
**Figure 4** is an illustration of the device according to the invention with the anti-return means being a configuration with the actuating means passing in and out the ligation as fastening means.
**Figures 5A, 5B and 5C** are illustrations of the device according to the invention with weld seams as anti-return means made integral with the actuating means, such weld seams being longitudinally spaced apart and separated by the fastening mean windings (5A), continuous and uniformly distributed along the actuating means (5B) and as a local reinforcement (5C).
**Figures 6A, 6B** and **6C** are three different illustrations of the device according to the invention with grooves built into the actuating means as anti-return means, such grooves being longitudinally spaced apart.
**Figures 7A** and **7B** are illustrations of the device according to the invention with a tube, crimped (7A) or glue filled (7B), as anti-return means.
**Figures 8A** and **8B** are illustrations of the device according to the invention showing an embodiment wherein the anti- return means and the actuating means are arranged alongside the internal periphery of the flexible elongated member. Fig. 8A shows the inner part of the flexible elongated member while Fig. 8B is an view from the outside.

### DETAILED DESCRIPTION

The following detailed description will be better understood when read in conjunction with the drawings. For the purpose of illustrating, the device according to the invention is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

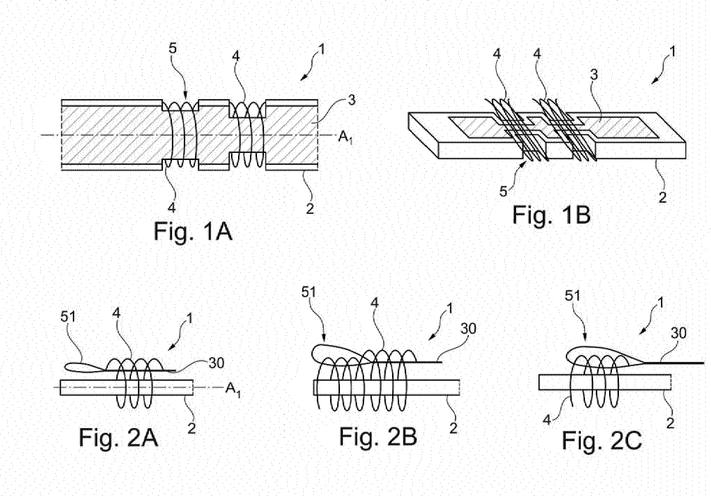

In **Figures 1A** and **1B****,** one can see an exemplary configuration of the device 1 according to the invention with recesses 5 in the flexible axially elongated member 2 as anti-return means in top and perspective views. In this figure, the flexible axially elongated member 2 is a blade, such blade has been made integral with the anti-return means in the form of two recesses 5. A blade is used in this embodiment, but a tubular element is also a possibility for the flexible, axially elongated member. The recesses 5 extend transversally towards the axis A1 of the blade 2. The actuating means is a flat wire 3 extending longitudinally while being in facial contact with the blade 2. It is thin compared to the ligation 4 diameter. Each of the two recesses 5 host ligations 4 as wires winding around said recesses 5 forming coil windings with their axis being the same as the axis A1 of elongation of the blade 2. The ligation 4 ties the flat wire 3 and fastens it to the blade 2 thanks to the grooves made in the actuating means 3. The combination of the ligation 4, the actuating mean grooves (1B) and potentially the recesses 5 allow to keep the flat wire 3 from sliding when actuated for instance by Joule effect when the flat wire 3 is a shape memory alloy such as NiTi type. In a preferred embodiment, the recess base of the flexible axially elongated member 2 and the groove base of the actuating means barely coincide radially as depicted in fig 1A.

In **figures 2A, 2B** and **2C****,** one can see an exemplary configuration of the device 1 according to the invention with loops 51 made integral with the wire 30 as anti-return means. In this figure, the flexible axially elongated member 2 is a blade but could have been a tubular element. In Figure **2A****,** the loop 51 serves as anti-return means. It is in axial abutment contact with the ligation 4 that ties the wire 30 to the blade 2. In Figure **2B****,** the loop 51 serves as anti-return means. It is in axial abutment contact with one part of the ligation 4 that ties the wire 30 to the blade 2 while also encapsulating i.e encompassing another part of the ligation 4 so as to improve the anti-return effect. In Figure **2C****,** the loop 51 serves as anti-return means. It is in axial abutment contact with the ligation 4 that ties the wire 30 to the blade 2 while also encapsulating i.e encompassing said ligation 4 so as to improve the anti-return effect. The windings of the ligation 4 are trapped within the close loop 51 made of the wire 30. The combination of the ligation 4 that ties the wire 30 to the blade 2 and the close loop 51 keeps the wire 30 from sliding when actuated for instance by Joule effect when the wire 30 is a shape memory alloy such as NiTi type.

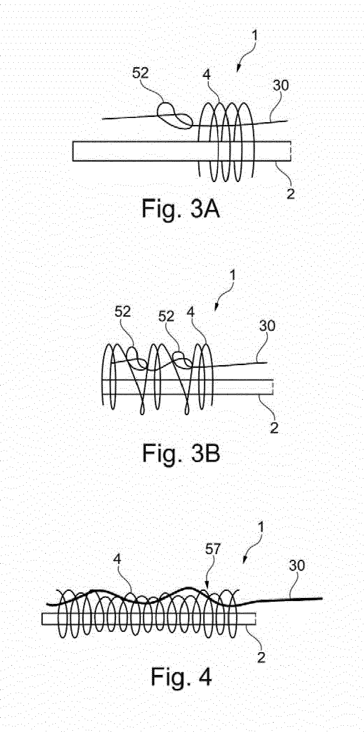

In **figures 3A** and **3B****,** one can see an exemplary configuration of the device 1 according to the invention with two knots 52 made integral with the wire 30 as anti-return means. In this figure, the flexible axially elongated member 2 is a blade but could have been a tubular element. In Figure **3A****,** there is only one knot and in figure **3B****,** there are two knots spaced apart longitudinally. the knot(s) 52 serve(s) as anti-return means. They are in axial abutment contact with the ligation 4 that ties the wire 30 to the blade 2.

In **figure 4****,** the anti-return means 57 is a configuration where the wire 30 and the ligation 4 are intertwined (not exactly illustrated for clarity purpose) with the wire 30 going alternatively up and under the windings of the ligation 4 so as to ligate longitudinally partial parts of the wire 30 with the flexible elongated member 2. This configuration increases the contact pressure between the actuating means 30 and the ligation 4, thus the adherence between the two latter is increased which is what creates the axial abutment. The ligation 4 ties the wire 30 to the blade 2 to keep the wire 30 from sliding when actuated for instance by Joule effect when the wire 30 is a shape memory alloy such as NiTi type.

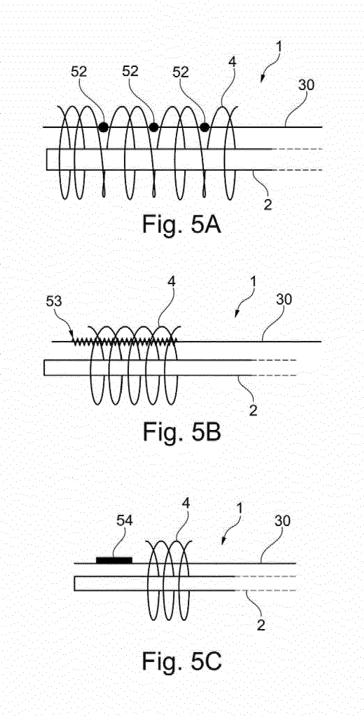

In **figures 5A, 5B** and **5C****,** one can see an exemplary configuration of the device 1 according to the invention with added material 52, 53 and 54 made integral with the wire 30 as anti-return means. In this figure, the flexible axially elongated member 2 is a blade but could have been a tubular element. In Figure **5A****,** the three weld seams serve as anti-return means. They are spaced apart longitudinally and in axial abutment contact with the ligation 4 windings that tie the wire 30 to the blade 2. In Figure **5B****,** the discontinuous weld spots 53 serve as anti-return means. They are in axial abutment contact with the ligation 4 windings that tie the wire 30 to the blade 2. In Figure **5C****,** the local reinforcement 54 serves as anti-return means, such local reinforcement 54 is axially offset in fig. 5C but could be located in between both ends of the ligation 4. The local reinforcement can be the result of a weld or not. Said local reinforcement 54 is in axial abutment contact with the ligation 4 windings that tie the wire 30 to the blade 2. The ligation 4 ties the wire 30 to the blade 2. The combination of the ligation and the anti-return means allows to keep the wire 30 from sliding when actuated for instance by Joule effect when the wire 30 is a shape memory alloy such as NiTi type.

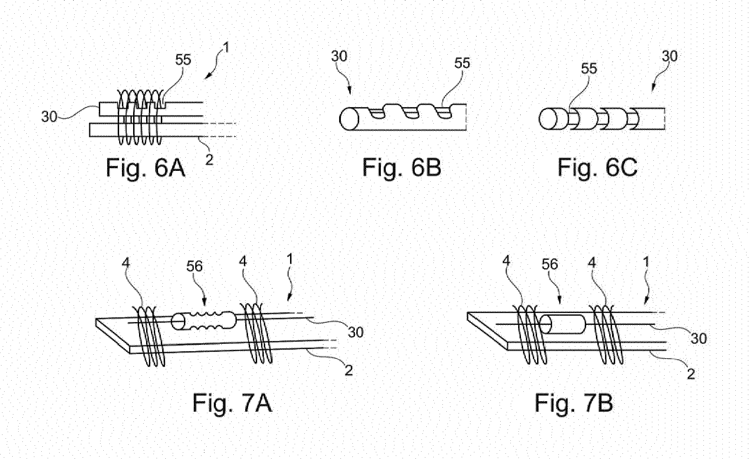

In **figures 6A, 6B** and **6C****,** one can see an exemplary configuration of the device 1 according to the invention with grooves 55 made integral with the wire 30 as anti-return means. In this figure, the flexible axially elongated member 2 is a blade but could have been a tubular element. In Figure **6A****,** the three grooves serve as anti-return means. They are spaced apart longitudinally and in axial abutment contact with the hosted ligation 4 windings that ties the wire 30 to the blade 2. Said grooves can be obtained by different processes such as for example indentation, pressure flattening, or even laser localized fusion. Figures **6B** and **6C** are illustrations of the grooves obtained on the wire with the different methods given as examples. The ligation 4 ties the wire 30 to the blade 2. The combination of the ligation and the at least one groove allows to keep the wire 30 from sliding when actuated for instance by Joule effect when the wire 30 is a shape memory alloy such as NiTi type.

Figures **7A** and **7B** are embodiments, where the anti-return means 56 is tube that is either crimped (Fig. 7A) or filled with glue (Fig. 7B). In both cases, the tube 56 comprises the wire 30 that goes through said tube 56. The glue or the crimping serves as a blocking element that holds the wire 30 in place. In Fig. 7A and 7B, the tube 56 has ligation 4 windings on both sides but a configuration with only one ligation 4 winding is possible as long as an axial abutment contact is obtained. The combination of the ligation 4 tying the wire 30 to the blade 2 in addition to the crimping or glue effect allows keeping the wire 30 from sliding when actuated for instance by Joule effect when the wire 30 is a shape memory alloy such as NiTi type.

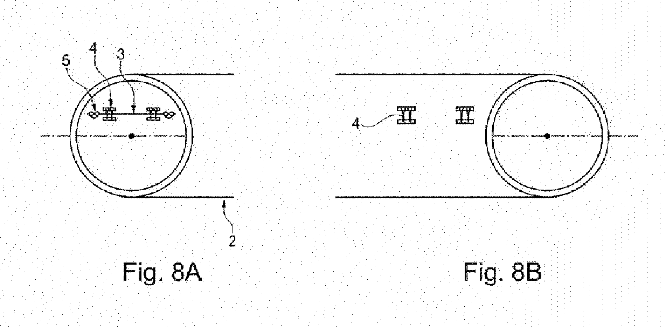

In **Figures 8A** and **8B****,** one can see a flexible elongated member in both internal (8A) and external (8B) views. As to Fig. 8A, an actuating means such as a shape memory alloy (SMA) wire 3 in anchored to the internal surface of the flexible elongated member 2. Ligation 4 windings are in axial abutting contact with the knot 5 so as to prevent the SMA wire 3 from sliding once actuated. The Fig. 8B is the view from outside showing ligation 4 windings going through recesses for the attachment.

While various embodiments have been described and illustrated, the detailed description is not to be construed as being limited hereto.

### REFERENCES

1 - steerable device
2 - flexible axially elongated member
3,30 - actuating means
4 - fastening means
5,51,52,53,54,55,56,57 - anti-return means

## Claims

1. A steerable device (1) configured to be advanced in the lumen of a tubular element, said device comprising:
- a flexible axially elongated member (2) having proximal and distal ends,
- at least one actuating means (3,30) arranged alongside the periphery of said elongated member (2),
- at least one fastening means (4) configured to fasten at least partially the at least one actuating means (3,30) to the flexible elongated member (2) distal end, said fastening means being in direct contact with the at least one actuating means (3,30),
- at least one anti-return means (5,51,52,53,54,55,56,57) configured to keep the at least one actuating means (3,30) from sliding alongside the periphery of the flexible elongated member (2) distal end,
**characterized in that** the at least one anti-return means (5,51,52,53,54,55,56,57) and the at least one fastening means (4) are in axial abutting contact so as to prevent the at least one actuating means (3,30) from sliding once actuated.

2. The steerable device according to claim **1,** wherein the at least one actuating means (3,30) is made of a shape memory alloy such as NiTi alloy.

3. The steerable device according to any one of claims **1** or **2,** wherein the at least one actuating means (3) comprises a spring or a wire (30).

4. The steerable device according to any one of claims **1** to **3,** wherein the at least one flexible axially elongated member (2) is wire or blade shaped.

5. The steerable device according to any one of claims **1** to **4,** wherein the flexible axially elongated member (2) has a cross-section profile under a form selected from star, circular, semicircular, square, rectangular, triangle, pyramidal or any combinations thereof.

6. The steerable device according to any one of claims **1** to **5,** wherein the fastening means (4) is a ligation around the flexible elongated member (2).

7. The steerable device according to any one of claims **1** to **6,** wherein the anti-return means (5) is a tube (56), either glue filled or crimped, the actuating means (3, 30) going through said tube (56).

8. The steerable device according to any one of claims **1** to **6,** further comprising a second anti-return means (5) made integral with the flexible elongated member (2) so as to maintain the fastening means (4) in place.

9. The steerable device according to claim **8,** wherein the anti-return means (5) is a recess built into the flexible elongated member (2) and configured to host the at least one fastening means (4).

10. The steerable device according to any one of claims **1** to **6** or **8** or **9,** wherein the anti-return means (51,52,53,54,55) is made integral with the actuating means (3,30).

11. The steerable device according to claim **10,** wherein the at least one anti-return means (5) is a loop (51) encapsulating at least partially the at least one fastening means (4) or said loop (51) being an abutment for the at least one fastening means (4).

12. The steerable device according to claim **10,** wherein the at least one anti-return means (5) is a knot (52), a weld (53) or any local reinforcement (54) located at the at least one actuating means (3,30) distal end.

13. The steerable device according to claim **10,** wherein the at least one anti-return means (5) is a groove (55) built into the actuating means (3,30) and configured to host the at least one fastening means (4).

14. The steerable device according to claim **13,** wherein the groove (55) is obtained by indentation.

15. The steerable device according to any one of claims **1** to **14** wherein the at least one actuating means (3,30) is inside an electrically isolating material such as a tube or a coating.

16. The steerable device according to any one of claims **1** to **15** wherein the at least one actuating means (3,30) is arranged alongside the external periphery of the elongated member (2).

17. Assembly method for steerable device according to any one of claims **1** to **16** comprising the steps of:
- providing a flexible axially elongated member (2) having proximal and distal ends,
- arranging at least one actuating means (3,30) alongside the periphery of said elongated member (2),
- providing at least one anti-return means (5) configured to keep the at least one actuating means (3,30) from sliding alongside the periphery of the flexible elongated member (2) distal end,
- fastening the at least one actuating means (3,30) to the flexible elongated member (2) distal end with at least one fastening means (4),
**characterized in that** the at least one anti-return means (5) and the at least one fastening means (4) are in axial abutting contact so as to prevent the at least one actuating means (3,30) from sliding once actuated.

18. Assembly method according to claim **17** wherein the at least one actuating means (3) is a wire (30), the at least one fastening means (4) is a ligation and the at least one anti-return means (57) is obtained by increasing the contact pressure between the wire (30) and the ligation (4) ,with said ligation going alternatively up and under the wire so as to ligate longitudinally partial parts of the wire with the flexible elongated member (2).

## Patentansprüche

1. Lenkbare Vorrichtung (1), die so konfiguriert ist, dass sie im Lumen eines rohrförmigen Elements vorgeschoben werden kann, wobei die Vorrichtung umfasst:
- ein flexibles, axial langgestrecktes Element (2), das ein proximales und ein distales Ende aufweist,
- mindestens ein Betätigungsmittel (3, 30), das entlang des Umfangs des langgestreckten Elements (2) angeordnet ist,
- mindestens ein Befestigungsmittel (4), das so konfiguriert ist, dass es das mindestens eine Betätigungsmittel (3, 30) mindestens teilweise am distalen Ende des flexiblen langgestreckten Elements (2) befestigt, wobei das Befestigungsmittel mit dem mindestens einen Betätigungsmittel (3, 30) in direktem Kontakt steht,
- mindestens ein Rücklaufsperrmittel (5, 51, 52, 53, 54, 55, 56, 57), das so konfiguriert ist, dass es das mindestens eine Betätigungsmittel (3, 30) davon abhält, entlang des Umfangs des distalen Endes des flexiblen langgestreckten Elements (2) zu gleiten,
**dadurch gekennzeichnet, dass** das mindestens eine Rücklaufsperrmittel (5, 51, 52, 53, 54, 55, 56, 57) und das mindestens eine Befestigungsmittel (4) in axialem Anschlagkontakt stehen, um zu verhindern, dass das mindestens eine Betätigungsmittel (3, 30) gleitet, sobald es betätigt wurde.

2. Lenkbare Vorrichtung nach Anspruch **1,** wobei das mindestens eine Betätigungsmittel (3, 30) aus einer Formgedächtnislegierung, wie etwa NiTi-Legierung, besteht.

3. Lenkbare Vorrichtung nach einem der Ansprüche **1** oder **2,** wobei das mindestens eine Betätigungsmittel (3) eine Feder oder einen Draht (30) umfasst.

4. Lenkbare Vorrichtung nach einem der Ansprüche **1** bis **3,** wobei das mindestens eine flexible, axial langgestreckte Element (2) draht- oder blattförmig ist.

5. Lenkbare Vorrichtung nach einem der Ansprüche **1** bis **4,** wobei das flexible, axial langgestreckte Element (2) ein Querschnittsprofil in einer Form aufweist, ausgewählt aus sternförmig, kreisförmig, halbkreisförmig, quadratisch, rechteckig, dreieckig, pyramidenförmig oder Kombinationen davon.

6. Lenkbare Vorrichtung nach einem der Ansprüche **1** bis **5,** wobei das Befestigungsmittel (4) eine Ligatur um das flexible langgestreckte Element (2) herum ist.

7. Lenkbare Vorrichtung nach einem der Ansprüche **1** bis **6,** wobei das Rücklaufsperrmittel (5) ein Rohr (56) ist, das entweder mit Klebstoff gefüllt oder verpresst ist, wobei das Betätigungsmittel (3, 30) durch das Rohr (56) verläuft.

8. Lenkbare Vorrichtung nach einem der Ansprüche **1** bis **6,** die weiter ein zweites Rücklaufsperrmittel (5) umfasst, das mit dem flexiblen langgestreckten Element (2) einstückig ausgebildet ist, um das Befestigungsmittel (4) an Ort und Stelle zu halten.

9. Lenkbare Vorrichtung nach Anspruch **8,** wobei das Rücklaufsperrmittel (5) eine Aussparung ist, die in das flexible langgestreckte Element (2) eingebaut und so konfiguriert ist, dass sie das mindestens eine Befestigungsmittel (4) aufnimmt.

10. Lenkbare Vorrichtung nach einem der Ansprüche **1** bis **6** oder **8** oder **9,** wobei das Rücklaufsperrmittel (51, 52, 53, 54, 55) mit dem Betätigungsmittel (3, 30) einstückig ausgebildet ist.

11. Lenkbare Vorrichtung nach Anspruch **10,** wobei das mindestens eine Rücklaufsperrmittel (5) eine Schlaufe (51) ist, die das mindestens eine Befestigungsmittel (4) mindestens teilweise einkapselt, oder wobei die Schlaufe (51) ein Anschlag für das mindestens eine Befestigungsmittel (4) ist.

12. Lenkbare Vorrichtung nach Anspruch **10,** wobei das mindestens eine Rücklaufsperrmittel (5) ein Knoten (52), eine Schweißnaht (53) oder eine lokale Verstärkung (54) ist, die sich an dem distalen Ende des mindestens einen Betätigungsmittels (3, 30) befindet.

13. Lenkbare Vorrichtung nach Anspruch **10,** wobei das mindestens eine Rücklaufsperrmittel (5) eine Nut (55) ist, die in das Betätigungsmittel (3, 30) eingebaut und so konfiguriert ist, dass sie das mindestens eine Befestigungsmittel (4) aufnimmt.

14. Lenkbare Vorrichtung nach Anspruch **13,** wobei die Nut (55) durch Einkerben erhalten wird.

15. Lenkbare Vorrichtung nach einem der Ansprüche **1** bis **14,** wobei sich das mindestens eine Betätigungsmittel (3, 30) innerhalb eines elektrisch isolierenden Materials, wie etwa einem Rohrs oder einer Ummantelung, befindet.

16. Lenkbare Vorrichtung nach einem der Ansprüche **1** bis **15,** wobei das mindestens eine Betätigungsmittel (3, 30) entlang des Außenumfangs des langgestreckten Elements (2) angeordnet ist.

17. Zusammenbauverfahren für eine lenkbare Vorrichtung nach einem der Ansprüche **1** bis **16,** das die folgenden Schritte umfasst:
- Bereitstellen eines flexiblen, axial langgestreckten Elements (2), das ein proximales und ein distales Ende aufweist,
- Anordnen mindestens eines Betätigungsmittels (3, 30) entlang des Umfangs des langgestreckten Elements (2),
- Bereitstellen mindestens eines Rücklaufsperrmittels (5), das so konfiguriert ist, dass es das mindestens eine Betätigungsmittel (3, 30) davon abhält, entlang des Umfangs des distalen Endes des flexiblen langgestreckten Elements (2) zu gleiten,
- Befestigen des mindestens einen Betätigungsmittels (3, 30) mit mindestens einem Befestigungsmittel (4) am distalen Ende des flexiblen langgestreckten Elements (2),
**dadurch gekennzeichnet, dass** das mindestens eine Rücklaufsperrmittel (5) und das mindestens eine Befestigungsmittel (4) in axialem Anschlagkontakt stehen, um zu verhindern, dass das mindestens eine Betätigungsmittel (3, 30) gleitet, sobald es betätigt wurde.

18. Zusammenbauverfahren nach Anspruch **17,** wobei das mindestens eine Betätigungsmittel (3) ein Draht (30) ist, das mindestens eine Befestigungsmittel (4) eine Ligatur ist und das mindestens eine Rücklaufsperrmittel (57) durch Erhöhen des Kontaktdrucks zwischen dem Draht (30) und der Ligatur (4) erhalten wird, wobei die Ligatur abwechselnd über und unter dem Draht verläuft, um teilweise Teile des Drahtes in Längsrichtung mit dem flexiblen langgestreckten Element (2) zu ligieren.

## Revendications

1. Dispositif orientable (1) configuré pour être avancé dans la lumière d'un élément tubulaire, ledit dispositif comprenant :
- un élément flexible et allongé axialement (2) ayant des extrémités proximale et distale,
- au moins un moyen d'actionnement (3,30) disposé le long de la périphérie dudit membre allongé (2),
- au moins un moyen de fixation (4) configuré pour fixer au moins partiellement l'au moins un moyen d'actionnement (3,30) à l'extrémité distale de l'élément flexible allongé (2), ledit moyen de fixation étant en contact direct avec l'au moins un moyen d'actionnement (3,30),
- au moins un moyen anti-retour (5,51,52,53,54,55,56,57) configuré pour empêcher l'au moins un moyen d'actionnement (3,30) de glisser le long de la périphérie de l'extrémité distale de l'élément flexible allongé (2),
**caractérisé en ce que** l'au moins un moyen anti-retour (5,51,52,53,54,55,56,57) et l'au moins un moyen de fixation (4) sont en contact axial de manière à empêcher l'au moins un moyen d'actionnement (3,30) de glisser une fois qu'il est actionné.

2. Dispositif orientable selon la revendication 1, dans lequel l'au moins un moyen d'actionnement (3,30) est constitué d'un alliage à mémoire de forme tel qu'un alliage NiTi.

3. Dispositif orientable selon l'une quelconque des revendications 1 ou 2, dans lequel l'au moins un moyen d'actionnement (3) comprend un ressort ou un fil (30).

4. Dispositif orientable selon l'une des revendications 1 à 3, dans lequel l'au moins un élément flexible axialement allongé (2) a la forme d'un fil ou d'une lame.

5. Dispositif orientable selon l'une des revendications 1 à 4, dans lequel l'élément flexible axialement allongé (2) a un profil de section transversale sous une forme choisie parmi une étoile, un cercle, un demi-circulaire, un carré, un rectangle, un triangle, une pyramide ou toute combinaison de celles-ci.

6. Dispositif orientable selon l'une des revendications 1 à 5, dans lequel le moyen de fixation (4) est une ligature autour de l'élément flexible allongé (2).

7. Dispositif orientable selon l'une quelconque des revendications 1 à 6, dans lequel le moyen anti-retour (5) est un tube (56), soit rempli de colle soit serti, le moyen d'actionnement (3, 30) passant à travers ledit tube (56).

8. Dispositif orientable selon l'une quelconque des revendications 1 à 6, comprenant en outre un second moyen anti-retour (5) rendu solidaire de l'élément flexible allongé (2) de manière à maintenir le moyen de fixation (4) en place.

9. Dispositif orientable selon la revendication 8, dans lequel le moyen anti-retour (5) est un renfoncement construit dans l'élément flexible allongé (2) et configuré pour accueillir l'au moins un moyen de fixation (4).

10. Dispositif orientable selon l'une quelconque des revendications 1 à 6 ou 8 ou 9, dans lequel le moyen anti-retour (51,52,53,54,55) est rendu solidaire du moyen d'actionnement (3,30).

11. Dispositif orientable selon la revendication 10, dans lequel l'au moins un moyen anti-retour (5) est une boucle (51) encapsulant au moins partiellement l'au moins un moyen de fixation (4) ou ladite boucle (51) étant une butée pour l'au moins un moyen de fixation (4).

12. Dispositif orientable selon la revendication 10, dans lequel l'au moins un moyen anti-retour (5) est un noeud (52), une soudure (53) ou tout renforcement local (54) situé à l'extrémité distale du au moins un moyen d'actionnement (3,30).

13. Dispositif orientable selon la revendication 10, dans lequel l'au moins un moyen anti-retour (5) est une rainure (55) intégrée au moyen d'actionnement (3,30) et configurée pour accueillir l'au moins un moyen de fixation (4).

14. Dispositif orientable selon la revendication 13, dans lequel la rainure (55) est obtenue par indentation.

15. Dispositif orientable selon l'une quelconque des revendications 1 à 14, dans lequel l'au moins un moyen d'actionnement (3,30) se trouve à l'intérieur d'un matériau isolant électriquement, tel qu'un tube ou un revêtement.

16. Dispositif orientable selon l'une des revendications 1 à 15, dans lequel l'au moins un moyen d'actionnement (3,30) est disposé le long de la périphérie externe de l'élément allongé (2).

17. Procédé d'assemblage d'un dispositif orientable selon l'une quelconque des revendications 1 à 16, comprenant les étapes suivantes:
- fournir un élément flexible axialement allongé (2) ayant des extrémités proximale et distale,
- disposer au moins un moyen d'actionnement (3,30) le long de la périphérie dudit élément allongé (2),
- fournir au moins un moyen anti-retour (5) configuré pour empêcher au moins un moyen d'actionnement (3,30) de glisser le long de la périphérie de l'extrémité distale de l'élément allongé flexible (2),
- fixer l'au moins un moyen d'actionnement (3,30) à l'extrémité distale de l'élément flexible allongé (2) à l'aide d'au moins un moyen de fixation (4),
**caractérisé en ce que** l'au moins un moyen anti-retour (5) et l'au moins un moyen de fixation (4) sont en contact axial avec la butée de manière à empêcher l'au moins un moyen d'actionnement (3,30) de glisser une fois actionné.

18. Procédé d'assemblage selon la revendication 17 dans lequel l'au moins un moyen d'actionnement (3) est un fil (30), l'au moins un moyen de fixation (4) est une ligature et l'au moins un moyen anti-retour (57) est obtenu en augmentant la pression de contact entre le fil (30) et la ligature (4), ladite ligature passant alternativement en haut et en bas du fil de manière à ligaturer longitudinalement des parties partielles du fil avec l'élément flexible allongé (2).
